(19) Europäisches Patentamt
European Patent Office
Office européen des brevets

(11) Veröffentlichungsnummer: **0 315 018**
**A2**

# (12) EUROPÄISCHE PATENTANMELDUNG

(21) Anmeldenummer: **88117702.6**

(22) Anmeldetag: **25.10.88**

(51) Int. Cl.4: **C07D 211/90 , C07D 401/12 , A61K 31/44**

(30) Priorität: **04.11.87 DE 3737340**

(43) Veröffentlichungstag der Anmeldung: **10.05.89 Patentblatt 89/19**

(84) Benannte Vertragsstaaten: **AT BE CH DE ES FR GB GR IT LI LU NL SE**

**POOR QUALITY**

(71) Anmelder: **BAYER AG**
**Konzernverwaltung RP Patentabteilung**
**D-5090 Leverkusen 1 Bayerwerk(DE)**

(72) Erfinder: **Franckowiak, Gerhard, Dr.**
**Henselweg 10**
**D-5600 Wuppertal 1(DE)**
Erfinder: **Stoltefuss, Jürgen, Dr.**
**Parkstrasse 20**
**D-5657 Haan(DE)**
Erfinder: **Bechem, Martin, Dr.**
**Obere Bergerheide 4**
**D-5600 Wuppertal 1(DE)**
Erfinder: **Gross, Rainer, Prof. Dr.**
**Platzhofstrasse 23**
**D-5600 Wuppertal 1(DE)**
Erfinder: **Kayser, Michael, Dr.**
**Walter-Flex-Strasse 18**
**D-5090 Leverkusen 1(DE)**
Erfinder: **Hebisch, Siegbert, Dr.**
**Herzogstrasse 129**
**D-4200 Oberhausen 11(DE)**
Erfinder: **Schramm, Matthias, Dr.**
**Paffrather Strasse 38**
**D-5000 Köln 80(DE)**

## (54) Fluormethoxyphenyl-dihydropyridine, Verfahren zur Herstellung und ihre Verwendung in Arzneimitteln.

(57) Die vorliegende Erfindung betrifft neue Fluormethoxyphenyl-dihydropyridine der allgemeinen Formel I

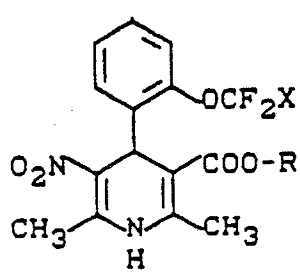

(I)

in welcher X und R die in der Beschreibung angegebene Bedeutung haben, mehrere Verfahren zu ihrer

EP 0 315 018 A2

Herstellung sowie ihre Verwendung in Arzneimitteln, insbesondere in kreislaufbeeinflussenden Mitteln mit positiv inotroper Wirkung.

# Neue Fluormethoxyphenyl-dihydropyridine, Verfahren zur Herstellung und ihre Verwendung in Arzneimitteln

Die vorliegende Erfindung betrifft neue Fluormethoxyphenyl-dihydropyridine, mehrere Verfahren zu ihrer Herstellung sowie ihre Verwendung in Arzneimitteln, insbesondere in kreislaufbeeinflussenden Mitteln mit positiv inotroper Wirkung.

Es ist bereits bekannt, daß 1,4-Dihydropyridine gefäßerweiternde Eigenschaften besitzen und als Coronarmittel und Antihypertensiva verwendet werden können (vgl. Brit. Patent 1 173 062 und 1 358 951; DE-OS 2 629 892 und 2 752 820). Weiterhin ist bekannt, daß 1,4-Dihydropyridine eine Hemmung der Kontraktionskraft von glatten und cardialen Muskeln bewirken und zur Behandlung von Coronar- und Gefäßerkrankungen eingesetzt werden können (vgl. Fleckenstein, Ann. Rev. Pharmcol. Toxicol. 17, 149 - 166 (1977)).

Bei Kenntnis dieser Eigenschaften der Dihydropyridine war es nicht vorhersehbar, daß die erfindungsgemäßen bindungen dieser Stoffklasse eine kontraktionskraftverstärkende, am Herzmuskel positiv inotrope Wirkung besitzen.

Die Erfindung betrifft neue Fluormethoxyphenyl-dihydropyridine der allgemeinen Formel (I),

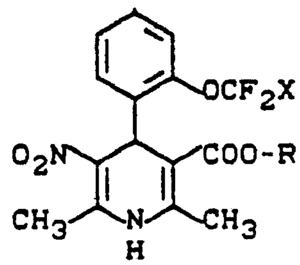

(I)

in welcher

X - für Wasserstoff oder Fluor steht und

R - für geradkettiges, verzweigtes oder cyclisches Alkyl oder Alkenyl mit 2 bis 12 Kohlenstoffatomen steht, das in der Kette durch 1 bis 2 Sauerstoff-und/oder Schwefelatome unterbrochen sein kann, und das ein- oder mehrfach gleich oder verschieden substituiert sein kann

a) durch Hydroxy, Aryl mit 6 bis 12 Kohlenstoffatomen, Aryloxy mit 6 bis 12 Kohlenstoffatomen, wobei der Arylrest wiederum durch Alkyl mit biz zu 4 Kohlenstoffatomen, Alkoxy mit bis zu 4 Kohlenstoffatomen oder Halogen substituiert sein kann, oder

b) durch Carboxy, Alkoxycarbonyl mit bis zu 6 Kohlenstoffatomen, Pyridyl, Piperidyl, Pyrimidyl, Acyloxy mit bis zu 7 Kohlenstoffatomen, Sulfamoyl, Carbamoyl, Halogen, Cyano-oder

c) durch eine Aminogruppe, wobei die Aminogruppe ein oder zwei gleiche oder verschiedene Substituenten der Reihe Alkyl mit bis zu 6 Kohlenstoffatomen, Benzyl, Phenyl oder Acyl mit bis zu 7 Kohlenstoffatomen tragen kann

in Form von Racematen und von optisch reinen Formen und deren Salzen.

Bevorzugt seien Verbindungen der allgemeinen Formel (I), in welcher

X - für Wasserstoff oder Fluor steht, und

R - für geradkettiges, verzweigtes oder cyclisches Alkyl mit 2 bis 12 Kohlenstoffatomen steht, das durch Sauerstoff und/oder Schwefel in der Kette unterbrochen sein kann, und das ein- bis zweimal gleich oder verschieden substituiert sein kann durch Hydroxy, Cyano, Phenyl, durch gegebenenfalls durch Methoxy, Methyl, Fluor oder Chlor substituiertes Phenoxy, durch Alkoxycarbonyl mit bis zu 6 Kohlenstoffatomen, durch Pyridyl, Piperidyl, Acetyloxy, Benzoyloxy oder durch eine Aminogruppe, wobei die Aminogruppe ein bis zwei gleiche oder verschiedene Substituenten der Reihe Alkyl mit bis zu 4 Kohlenstoffatomen oder Benzyl, tragen kann, oder

- für geradkettiges oder verzweigtes Alkenyl mit bis zu 10 Kohlenstoffatomen steht

und deren Salze.

Besonders bevorzugt seien Verbindungen der allgemeinen Formel (I) genannt, in welcher

X - für Wasserstoff oder Fluor steht, und

R - für geradkettiges oder verzweigtes Alkyl mit 2 bis 12 Kohlenstoffatomen steht, das in der Kette durch

ein Sauerstoffatom oder ein Schwefelatom unterbrochen sein kann und das ein- oder zweifach gleich oder verschieden substituiert sein kann durch Hydroxy, Cyano, Phenyl, Phenoxy, 4-Methoxyphenoxy, Pyridyl, Alkoxycarbonyl mit bis zu 4 Kohlenstoffatomen, Amino oder Benzylmethylamino oder
- für geradkettiges oder verzweigtes Alkenyl mit bis zu 10 Kohlenstoffatomen steht, oder
- für Cyclopropyl, Cyclopentyl, Cyclohexyl, Cyclopropylmethyl, Cyclopentylmethyl oder Cyclohexylmethyl steht

sowie deren Salze.

Physiologisch unbedenkliche Salze können Salze der erfindungsgemäßen Verbindungen mit anorganischen oder organischen Säuren sein. Als Beispiele seien genannt: Hydrohalogenide wie zum Beispiel Hydrochloride oder Hydrobromide, Hydrogensulfate, Sulfate, Hydrogenphosphate, Phosphate, oder Acetate, Maleate, Fumarate, Citrate, Tartrate, Lactate oder Benzoate.

Die erfindungsgemäßen Verbindungen der Formel (I) in racemischer Form, in welcher
R die oben angegebene Bedeutung hat,
erhält man, indem man

[A] Aldehyde der Formel (II)

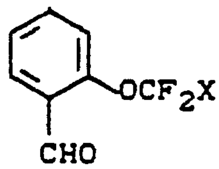

(II)

in dem X die oben angegebene Bedeutung hat,
und Nitroaceton der Formel (III)

$H_3C\text{-}CO\text{-}CH_2\text{-}NO_2$ (III)

oder deren Knoevenagel-Kondensationsprodukt der Formel (IV)

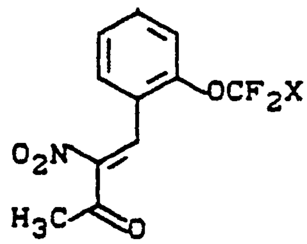

(IV)

in dem X die oben angegebene Bedeutung hat,
und Aminocrotonsäureester der allgemeinen Formel (V)

$$H_3C\text{-}\underset{\displaystyle NH_2}{\underset{|}{C}}=CH\text{-}COOR \qquad (V)$$

in welcher
R die oben angegebene Bedeutung hat,,
gegebenenfalls in Anwesenheit inerter Lösemittel umsetzt,
oder indem man

[B] das Dihydropyridin-Derivat der Formel (VI)

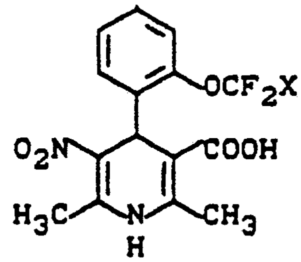

(VI)

in dem X die oben angegebene Bedeutung hat,
mit Alkoholen der Formel (VII)

R-OH (VII)

in welcher
R die oben angegebene Bedeutung hat,
nach bekannten Methoden, gegebenenfalls über ein reaktives Säurederivat, umsetzt,
oder indem man

[C] Aldehyde der Formel (II) und Acetessigester der Formel (VIII)

$H_3C\text{-}CO\text{-}CH_2\text{-}COOR$ (VIII)

bzw. deren Knoevenagel-Kondensationsprodukte der Formel (IX)

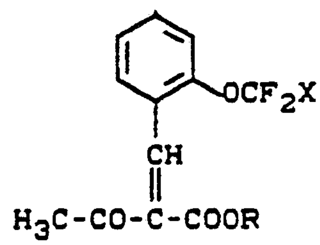

(IX)

in denen X und R die oben angegebenen Bedeutungen haben,
mit Nitroaceton und Ammoniumsalzen wie z.B. Ammoniumacetat, gegebenenfalls in Anwesenheit inerter Lösemittel, umsetzt, und gegebenenfalls die erhaltenen Dihydropyridine nach üblichen Methoden in ihre optisch aktiven Isomere überführt.

Enantiomerenreine Formen erhält man z.B., indem man Diastereomerengemische von Dihydropyridinen der Formel (X)

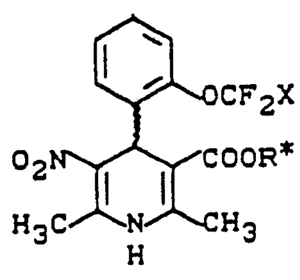

(X)

in welcher
X die oben angegebene Bedeutun hat, und
R* einen optisch aktiven Esterrest darstellt,
durch Kristallisation, Chromatographie oder Craig-Verteilung in die einzelnen Diastereomeren trennt, anschließend die enantiomerenreinen Carbonsäuren der Formel XI

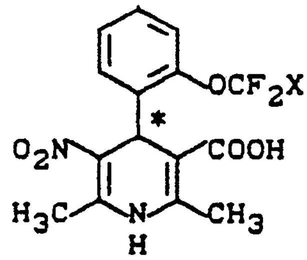

(XI)

hersteilt,
und dann diese gegebenenfalls durch Veresterung mit Alkoholen der allgemeinen Formel (VII) in die entsprechenden enantiomerenreinen Dihydropyridine der Formel (I) umsetzt.

Je nach Art der verwendeten Ausgangsstoffe läßt sich die Synthese der racemischen Verbindungen nach Verfahren A bis C durch folgende Formelschemata verdeutlichen:

[A]

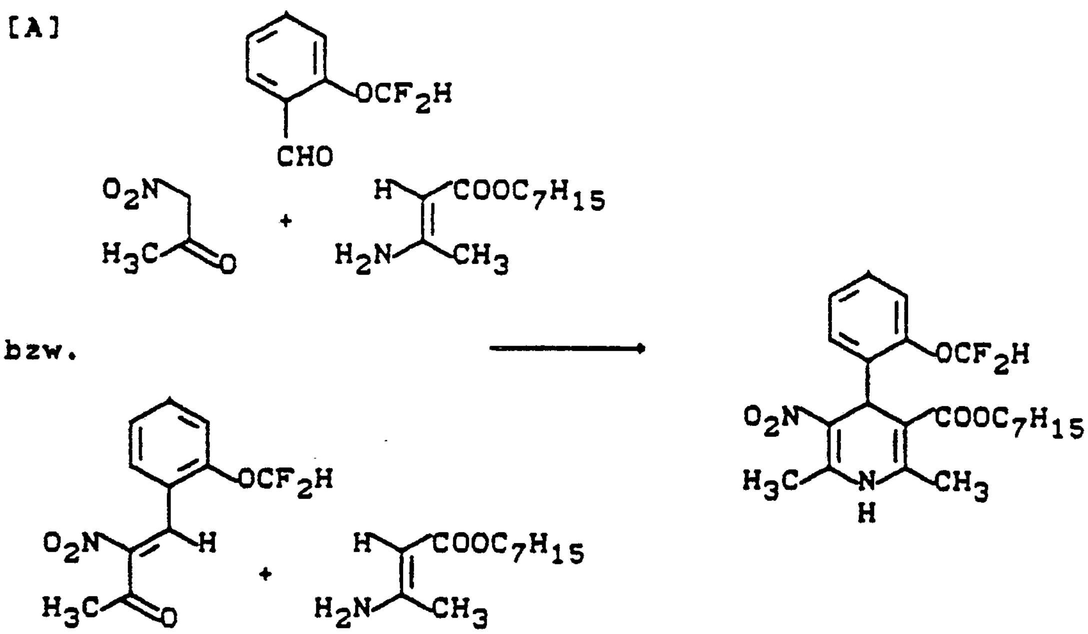

bzw.

[B]

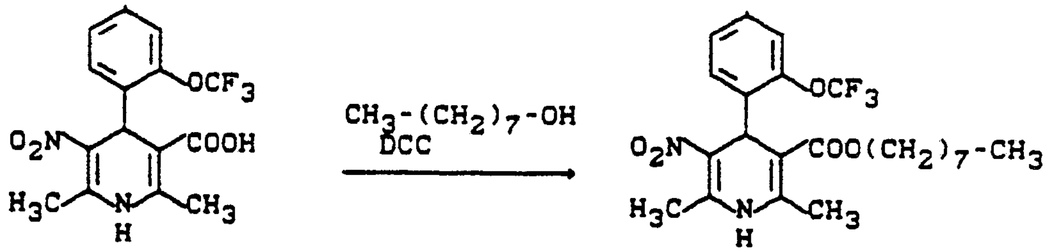


[C]

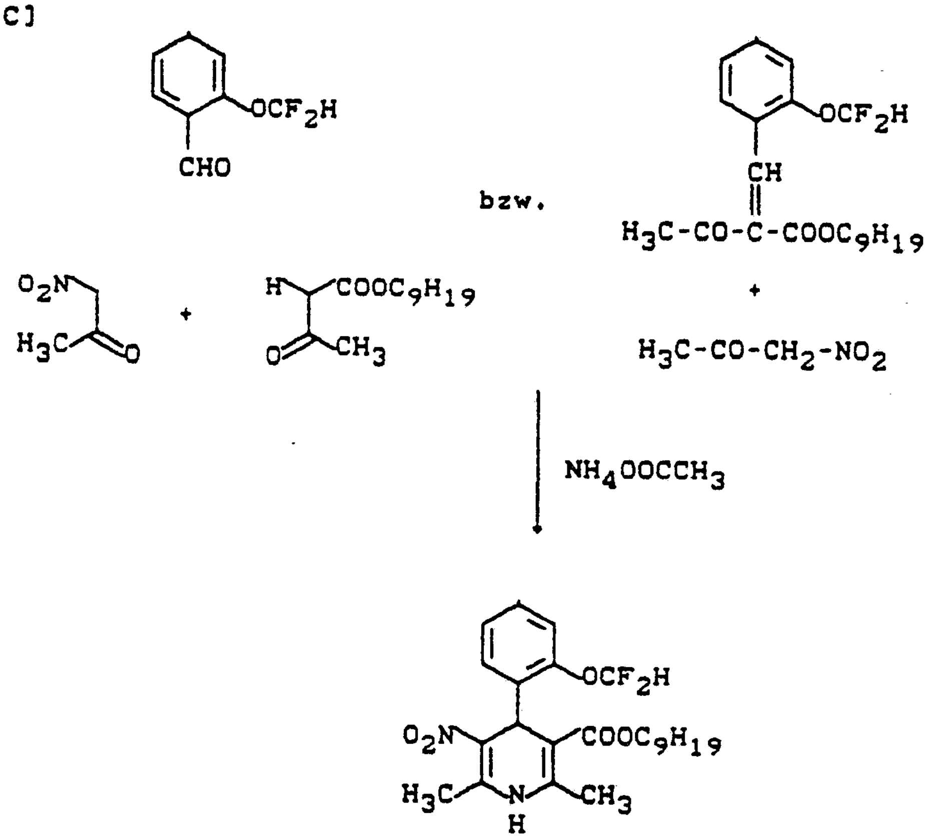


Die Enantiomerentrennung kann z.B. durch folgendes Formelschema verdeutlicht werden:

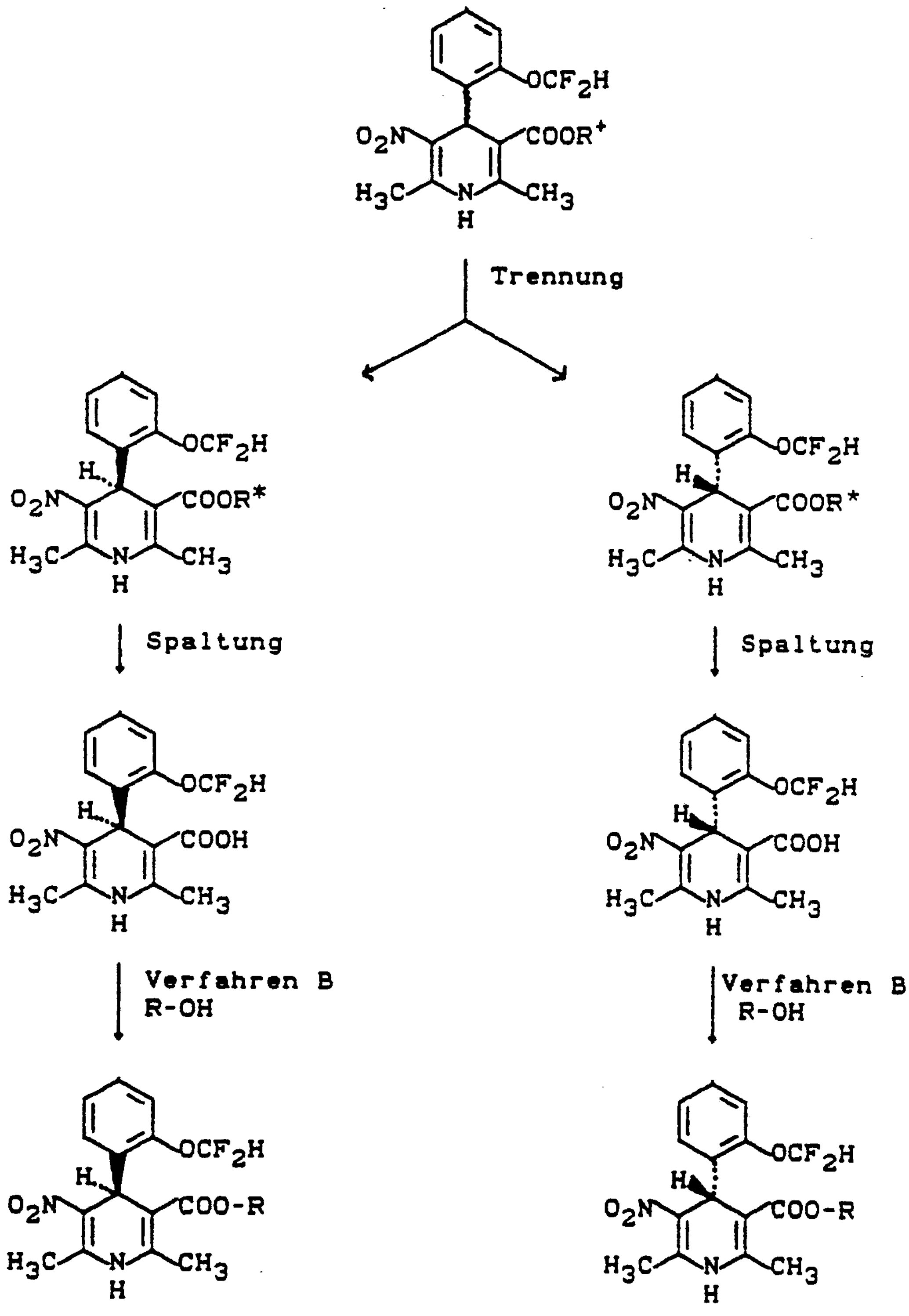


Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Geeignet als chirale Esterreste sind alle Ester enantiomerenreiner Alkohole wie beispielsweise 2-Butanol, 1-Phenylethanol, Milchsäure, Milchsäureester, Mandelsäure, Mandelsäureester, 2-Aminoalkohole, Zuckerderivate und viele andere enantiomerenreine Alkohole mehr.

Die Trennung der Diastereomeren erfolgt im allgemeinen entweder durch fraktionierte Kristallisation,

durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Als Lösungsmittel verwendet man die für solche Prozeduren üblichen Lösungsmittel wie bevorzugt Alkohole wie Methanol, Ethanol, n- bzw. iso-Propanol, Ether wie Diethylether, Tetrahydrofuran, Dioxan oder Glykolmono-oder -diethylether, Eisessig, Pyridin, Dimethylformamid, Dimethylsulfoxid, Acetonitril, Hexamethylphosphorsäuretriamid, Toluol, Aceton, Methylenchlorid, Hexan, Formamid, Wasser, Essigester usw..

Die Abspaltung der chiralen Estergruppen kann je nach Art des chiralen Esters durch saure oder alkalische Hydrolyse oder durch $\beta$-Eliminierung in den für solche Prozeduren üblichen Lösungsmitteln erfolgen.

Die Temperaturen können in einem weiten Bereich variiert werden. Bevorzugt sind Temperaturen zwischen 20° C und 100° C.

Die Veresterung der enantiomerenreinen Dihydropyridincarbonsäuren der Formel XI mit den Alkoholen der Formel VII zu den enantiomerenreinen Verbindungen der Formel I erfolgt nach bekannten Methoden, gegebenenfalls über ein reaktives Säurederivat nach den üblichen Methoden der Veresterung in den üblichen Lösungsmitteln wie bevorzugt Ether wie Diethylether oder Tetrahydrofuran, Dimethylformamid, Methylenchlorid, Chloroform, Acetonitril, Toluol usw..

Bevorzugte Temperaturen der Veresterung sind Temperaturen zwischen 0° C und 100° C.

Darüber hinaus ist es möglich, die enantiomerenreinen Carbonsäuren durch Trennung der racemischen Carbonsäuren zu gewinnen und diese dann, wie in Verfahren B beschrieben, mit Alkoholen der Formel VII umzusetzen.

Die als Ausgangsstoffe verwendeten Fluormethoxybenzaldehyde können nach in der Fluorchemie üblichen Methoden aus Salicylaldehyd hergestellt werden.

Nitroaceton(III) ist bekannt [N. Levy, C.W. Scaife, J. Chem. Soc. (London) 1946, 1100, C.D. Hurd, M.E. Nilson, J. Org. Chem. 20, 927 (1955)].

Die Ylidenverbindungen IV sind neu, können aber nach bekannten Methoden hergestellt werden (vgl. H. Dornow und W. Sassenberg, Liebigs Ann. Chem. 602, 14 (1957)).

Die Aminocrotonsäureester der Formel V sind bekannt oder können nach bekannten Methoden hergestellt werden [S.A. Glickman, A.C. Cope, J. Am. Chem. Soc. 67, 1017 (1945)].

Die erfindungsgemäßen Acetessigester der Formel VIII sind bekannt oder können nach bekannten Methoden hergestellt werden [vgl. D. Borrmann, "Umsetzung von Diketen mit Alkoholen, Phenolen und Mercaptanen", in Houben-Weyl, Methoden der organischen Chemie, Vol. VII/4, 230 ff (1968)].

Die erfindungsgemäß verwendbaren Ylidenverbindungen der Formel IX sind bekannt oder können nach bekannten Methoden hergestellt werden [Organic Reactions XV, 204 ff (1967)].

Die racemischen DHP-Carbonsäuren VI sind neu, können aber nach bekannten Methoden hergestellt werden [vgl. EP-71 819].

Die Verbindungen der Formel I, in welcher R für einen chiralen Rest steht, lassen sich nach den Verfahren A bis C herstellen.

Als Verdünnungsmittel kommen für Verfahren A bis C alle inerten organischen Lösemittel in Frage. Hierzu gehören bevorzugt Alkohole wie Methanol, Ethanol, n- bzw. iso-Propanol, Butanol, Ether wie Diethylether, Tetrahydrofuran, Dioxan oder Glykolmono- oder diethylether, Eisessig, Pyridin, Dimethylformamid, Dimethylsulfoxid, Acetonitril, Hexamethylphosphorsäuretriamid oder Toluol.

Die Reaktionstemperaturen für Verfahren A bis C können in einem größeren Bereich variiert werden. Im allgemeinen arbeitet man in einem Bereich von 10° C bis 200° C, bevorzugt von 20° C bis 150° C.

Bei der Durchführung der erfindungsgemäßen Verfahren ist das Verhältnis der an der Reaktion beteiligten Stoffe beliebig. Im allgemeinen arbeitet man jedoch mit molaren Mengen der Reaktanden.

Die vorstehenden Herstellungsverfahren sind lediglich zur Verdeutlichung angegeben. Die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur. Sie können deshalb in Arzneimitteln zur Beeinflussung des pathologisch veränderten Blutdrucks, als Koronartherapeutika und zur Behandlung der Herzinsuffizienz eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die kurzkettigen Alkylester der erfindungsgemäßen Verbindungen (insbesondere solche mit $R<C_6$) zeigen zum Teil eine sehr starke positiv inotrope Wirkung, die jedoch gleichzeitig mit einer Gefäßkonstriktionswirkung verbunden sein kann.

Die Herz- und Gefäßwirkungen wurden am isoliert perfundierten Herzen des Meerschweinchens gefunden. Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. Als Perfusionsmedium dient eine Krebs-Henseleit-Lösung (1) (118,5 mmol/l NaCl, 4,75 mmol/l KCl, 1,19 mmol/l $KH_2PO_4$, 1,19 mmol/l $MgSO_4$, 25 mmol/l $NaHCO_3$, 0,013 mmol/l $Na_2EDTA$), deren $CaCl_2$-Gehalt 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32° C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert (Opie, L., J. Physiol. 180 (1965), 529 -541). Der Perfusionsdruck wird mittels eines Druckaufnehmers, der vor dem Herzen mit dem Perfusionssystem in Verbindung steht, registriert. Unter diesen Bedingungen zeigt eine Senkung des Perfusionsdrucks eine Koronardilatation, eine Zu- bzw. Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Die folgenden Werte zeigen beispielhaft den Effekt der erfindungsgemäßen Verbindungen am isoliert perfundierten Meerschweinchenherzen, ausgedrückt als prozentuale Differenz gegenüber dem gleich 100% gesetzten Ausgangswert.

| Bsp.-Nr. | Konzentration (g/l) | %-Änderung der Ventrikeldruckamplitude |
|---|---|---|
| 11 | $10^{-3}$ | + 45 |
| 12 | $10^{-3}$ | + 45 |
| 25 | $10^{-3}$ | + 25 |
| 27 | $10^{-3}$ | + 25 |
| 28 | $10^{-3}$ | + 56 |
| 36 | $10^{-3}$ | + 39 |
| 44 | $10^{-3}$ | + 40 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt : Wasser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten

Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Experimenteller Teil

Beispiel 1

4-(2-Difluormethoxyphenyl)-1,4-dihydro-2,6-dimethyl-3-nitro-pyridin-5-carbonsäure-(3-phenylpropyl)-ester

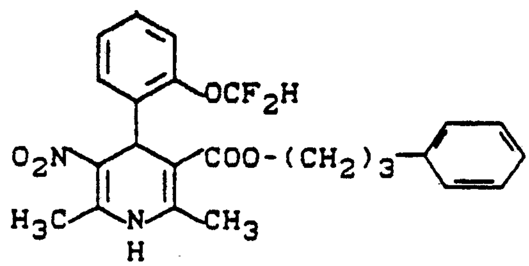

Verfahrensvariante A

3,4 g (20 mmol) 2-Difluormethoxybenzaldehyd werden in 30 ml Ethanol mit 3,6 g (35 mmol) Nitroaceton, 4,4 g (20 mmol) β-Aminocrotonsäure-(3-phenylpropyl)-ester und 1,2 ml (20 mmol) Essigsäure 4 Stunden gekocht. Es wird abgekühlt und eingeengt. Der ölige Eindampfrückstand wird in Essigester gelöst, mit Wasser, Natriumhydrogencarbonatlösung und wieder Wasser gewaschen, getrocknet und eingeengt. Das erhaltene ölige Rohprodukt wird über eine Kieselgelsäule von 360 ml Volumen mit Toluol/Essigester gereinigt. Das erhaltene Öl kristallisiert beim Verrühren mit Ether. Es wird abgesaugt und mit Ether gewaschen. Man erhält 2,2 g (24% der Theorie) gelbe Kristalle vom Schmelzpunkt 162-164 °C.

Beispiel 2

4-(2-Difluormethoxyphenyl)-1,4-dihydro-2,6-dimethyl-3-nitro-pyridin-5-carbonsäure-n-butylester

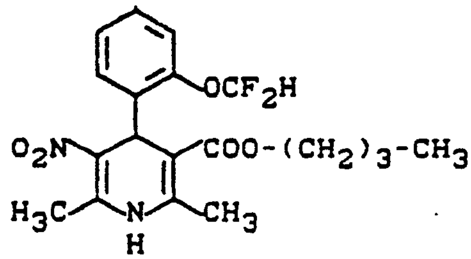

Verfahrensvariante A

3,86 g (15 mmol) 2-Difluormethoxybenzyliden-nitroaceton werden in 25 ml Ethanol mit 2,4 g (15 mmol) β-Aminocrotonsäurebutylester und 0,9 ml (15 mmol) Essigsäure 4 Stunden gekocht. Es wird abgekühlt und eingeengt. Der ölige Eindampfrückstand wird in Essigester aufgenommen, mit Wasser, Natriumhydrogencarbonatlösung und wieder Wasser gewaschen, getrocknet und eingeengt. Der ölige Rückstand kristallisiert unter Hexan nach Zugabe von wenig Ether. Es wird abgesaugt und mit Hexan/Ether im Volumenverhältnis 10:1 gewaschen. Man erhält 5,1 g (85,9% der Theorie) orangegelbe Kristalle vom Schmelzpunkt 118-120° C.

Beispiel 3

Herstellung reiner Enantiomere über chirale Ester

4-(2-Difluormethoxyphenyl)-1,4-dihydro-2,6-dimethyl-3-nitro-pyridin-5-carbonsäure-1(S)-(1-isopropoxycarbonylethyl)-ester.

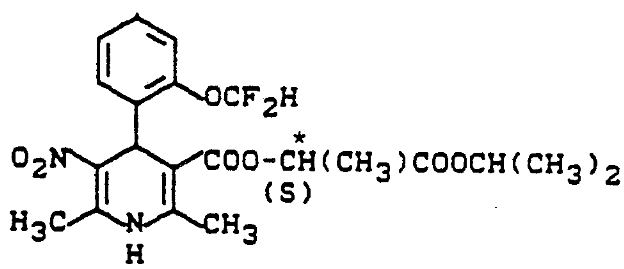

5,14 g (20 mmol) 2-Difluormethoxybenzyliden-nitroaceton werden in 25 ml Ethanol mit 4,3 g (20 mmol) β-Aminocrotonsäure-1(S)-(1-isopropoxycarbonyl-ethyl)-ester und 1,2 ml (20 mmol) Eisessig 4 Stunden gekocht. Es wird eingeengt, in Essigester gelöst und mit Wasser, Natriumhydrogencarbonat-Lösung und wieder Wasser gewaschen, getrocknet und eingeengt. Der erhaltene Eindampfrückstand wird in wenig Ether gelöst und stehen gelassen, wobei ein Diastereomeres auskristallisiert. Es wird abgesaugt und mit Ether gewaschen. Man erhält 2,13 g (23,5 %) gelb gefärbte Kristalle vom Schmelzpunkt 167-169° C.

Beispiel 4

4-(2-Difluormethoxyphenyl)-1,4-dihydro-2,6-dimethyl-3-nitropyridin-5-carbonsäure ((-)-Enantiomer)

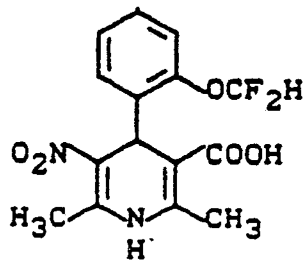

1,14 g (2,5 mmol) der nach Beispiel 3 erhaltenen Kristalle werden in 40 ml Dioxan und 50 ml 1,5 N Natronlauge 48 Stunden gerührt. Es wird etwas eingeengt und 2 x mit Methylenchlorid ausgeschüttelt. Die wäßrige Phase wird mit 10 %iger Salzsäure angesäuert, 2 x mit Essigester ausgeschüttelt, mit Wasser gewaschen, getrocknet und eingeengt. Das Produkt wurd über eine kurze Kieselgelsäule gereinigt. Man erhält 245 mg (28,8 %) gelb gefärbten Schaum von Drehwert

$[\alpha]^{20}_{589}$ = - 17,6 (c = 0,512, Aceton).

Beispiel 5

Analog Beispiel 4 wird aus dem anderen Diastereomeren die (+) 4-(2-Difluormethoxyphenoxy)-1,4-dihydro-2,6-dimethyl-3-nitropyridin-5-carbonsäure erhalten

$[\alpha]^{20}_{589}$ = + 20,4 (c = 1,023 Aceton)

Beispiel 6

Verfahrensvariante B

4-(2-Difluormethoxyphenyl)-1,4-dihydro-2,6-dimethyl-3-nitro-pyridin-5-carbonsäure-(6-hydroxyhexyl)-ester ((-)-Enantiomeres)

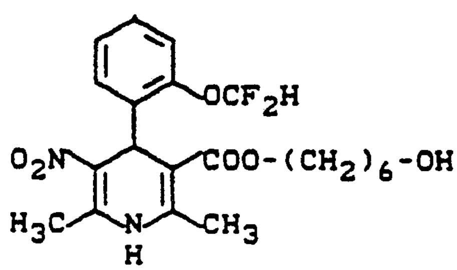

1,5 g (4,4 mmol) der Verbindung aus Beispiel 5 werden in 10 ml absolutem Dimethylformamid gelöst und nacheinander mit 10 g Hexandiol-1,6, 0,44 g 4-Dimethylaminopyridin und 2,55 g (6 mmol) 1-Cyclohexyl-3-(2-morpholinoethyl)-carbodiimid-p-methoxy-toluolsulfonat 44 Stunden gerührt. Es wird vom ausgefallenen Salz abgesaugt, das Filtrat wird eingeengt, der ölige Rückstand in Essigester aufgenommen, mit Wasser, ca. 5%iger Salzsäure, Wasser, Natriumhydrogencarbonatlösung und wieder Wasser gewaschen, getrocknet und eingeengt. Der Eindampfrückstand wird durch Flash-Chromatographie mit Cyclohexan/Toluol-Gemischen gereinigt. Die reinen Fraktionen werden eingeengt, durch Verrühren mit Ether kristallisiert, abgesaugt und mit Ether gewaschen. Man erhält 1,4 g (72% der Theorie) gelbe Kristalle vom Schmelzpunkt 122-124° C.

$[\alpha]^{20}_{589}$ = -24,64 (c=0,9365, Chloroform)

Analog Beispiel 1 bis 3 wurden hergestellt:

Beispiel 7

4-(2-Difluormethoxyphenyl)-1,4-dihydro-2,6-dimethyl-3-nitro-pyridin-5-carbonsäure-ethylester

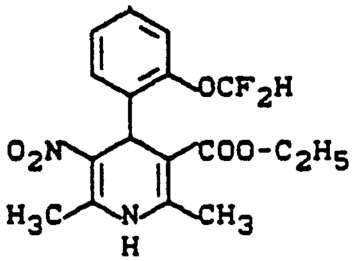

Schmp.: 92° C

Beispiel 8

4-(2-Difluormethoxyphenyl)-1,4-dihydro-2,6-dimethyl-3-nitro-pyridin-5-carbonsäure-isopropylester

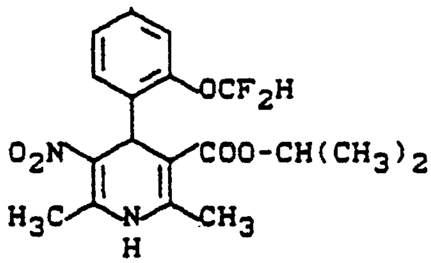

Schmp.: Harz

Beispiel 9

4-(2-Difluormethoxyphenyl)-1,4-dihydro-2,6-dimethyl-3-nitro-pyridin-5-carbonsäure-(β-cyanoethyl)-ester

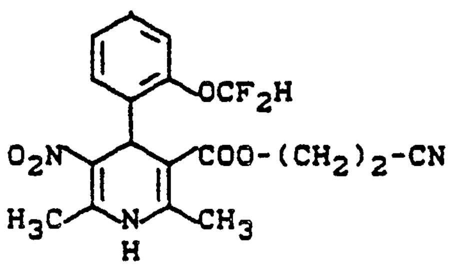

Schmp.: 150° C

Beispiel 10

4-(2-Difluormethoxyphenyl)-1,4-dihydro-2,6-dimethyl-3-nitro-pyridin-5-carbonsäure-(3,3-dimethylbutyl)-ester

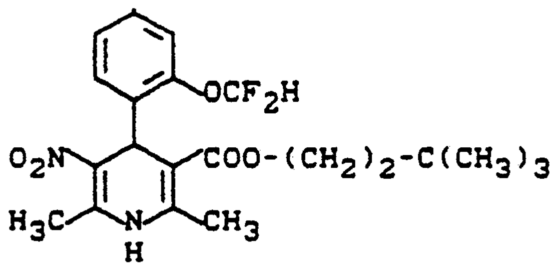

Schmp.: Harz

Beispiel 11

4-(2-Difluormethoxyphenyl)-1,4-dihydro-2,6-dimethyl-3-nitro-pyridin-5-carbonsäure-(2-acetoxyethyl)-ester

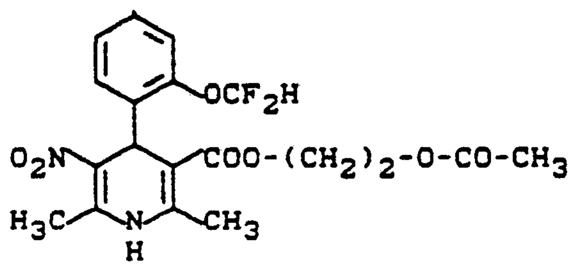

Schmp. : 137° C

Beispiel 12

4-(2-Difluormethoxyphenyl)-1,4-dihydro-2,6-dimethyl-3-nitro-pyridin-5-carbonsäure-(2-N-piperidinyl-ethyl)-ester

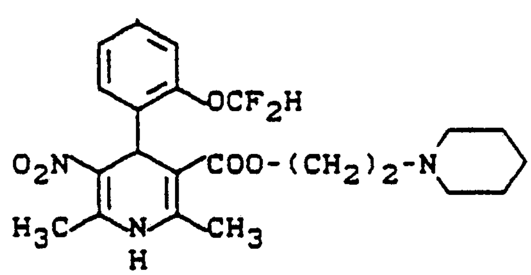

Schmp.: Harz

Beispiel 13

4-(2-Difluormethoxyphenyl)-1,4-dihydro-2,6-dimethyl-3-nitro-pyridin-5-carbonsäure-(2-(N-benzyl-N-methyl)-aminoethyl)-ester

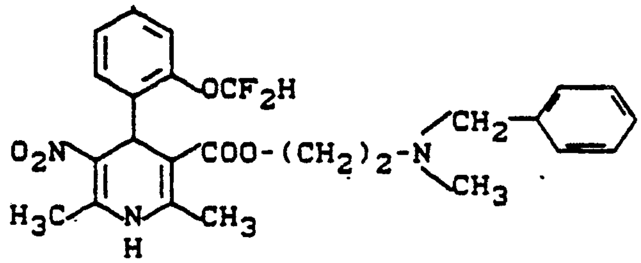

Schmp.: Harz

Beispiel 14

4-(2-Difluormethoxyphenyl)-1,4-dihydro-2,6-dimethyl-3-nitro-pyridin-5-carbonsäure-(2-(2-pyridyl)-ethyl)-ester

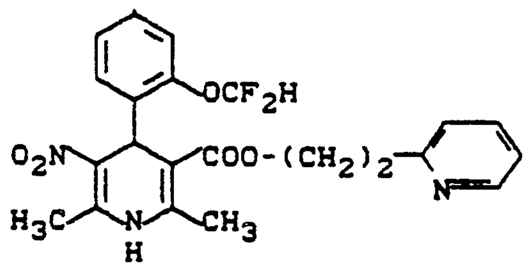

Schmp.: 159° C

Beispiel 15

4-(2-Difluormethoxyphenyl)-1,4-dihydro-2,6-dimethyl-3-nitro-pyridin-5-carbonsäure-octylester

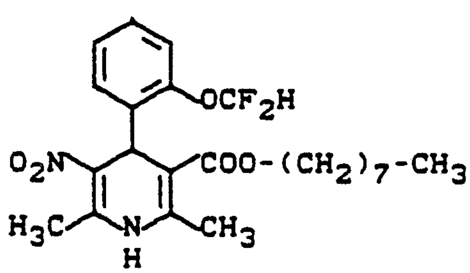

Schmp.: 99 - 100° C

Beispiel 16

4-(2-Difluormethoxyphenyl)-1,4-dihydro-2,6-dimethyl-3-nitro-pyridin-5-carbonsäure-(2-(4-methoxy-phenoxy)-ethyl)-ester

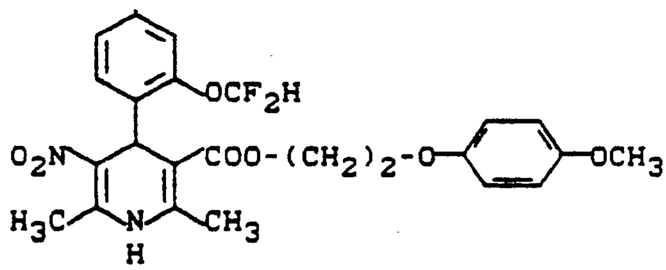

Schmp.: 115 - 118° C

Beispiel 17

4-(2-Difluormethoxyphenyl)-1,4-dihydro-2,6-dimethyl-3-nitro-pyridin-5-carbonsäure-dodecylester

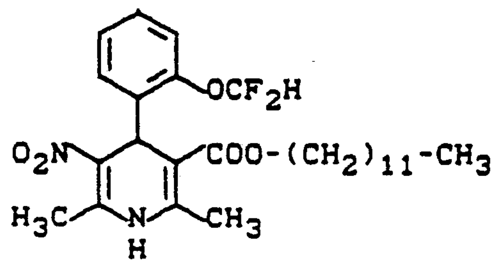

Schmp.: 88-90° C

Beispiel 18

4-(2-Difluormethoxyphenyl)-1,4-dihydro-2,6-dimethyl-3-nitro-pyridin-5-carbonsäure-(3-phenoxypropyl)-ester

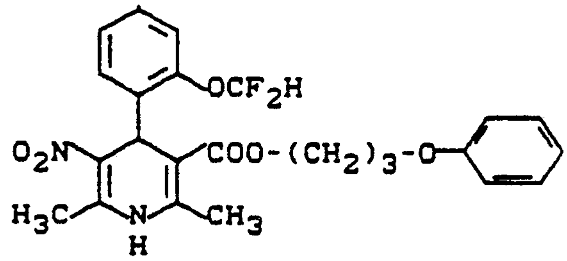

Schmp.: 146 - 149° C

Beispiel 19

4-(2-Difluormethoxyphenyl)-1,4-dihydro-2,6-dimethyl-3-nitro-pyridin-5-carbonsäure-(2-phenylethyl)-ester

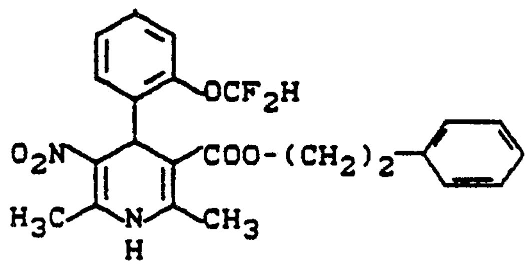

Schmp.: 123° C

Beispiel 20

4-(2-Difluormethoxyphenyl)-1,4-dihydro-2,6-dimethyl-3-nitro-pyridin-5-carbonsäure-(5-phenylpentyl)-ester

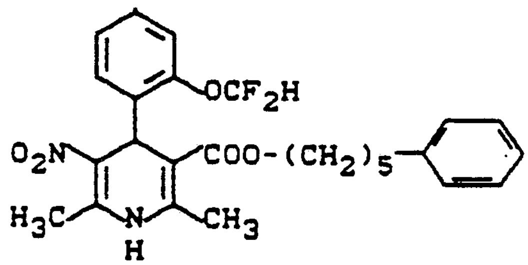

Schmp.: 88-90° C

Beispiel 21

4-(2-Difluormethoxyphenyl)-1,4-dihydro-2,6-dimethyl-3-nitro-pyridin-5-carbonsäure-nonylester

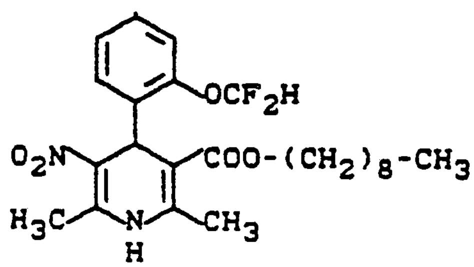

Schmp.: 96 - 97° C

Beispiel 22

4-(2-Difluormethoxyphenyl)-1,4-dihydro-2,6-dimethyl-3-nitro-pyridin-5-carbonsäure-(4-phenylbutyl)-ester

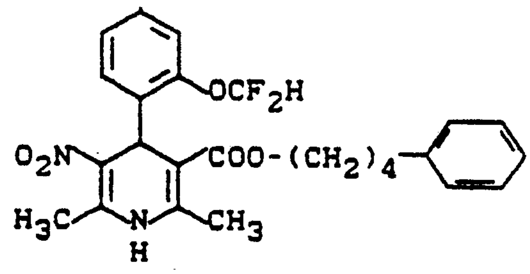

Schmp.: ab 106° C

Beispiel 23

4-(2-Difluormethoxyphenyl)-1,4-dihydro-2,6-dimethyl-3-nitro-pyridin-5-carbonsäure-heptylester

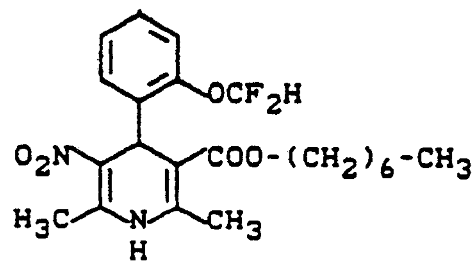

Schmp.: 78 - 80° C

Beispiel 24

4-(2-Difluormethoxyphenyl)-1,4-dihydro-2,6-dimethyl-3-nitro-pyridin-5-carbonsäure-hexylester

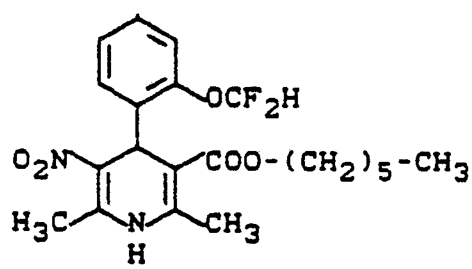

Schmp.: 98 - 100° C

Beispiel 25

4-(2-Difluormethoxyphenyl)-1,4-dihydro-2,6-dimethyl-3-nitro-pyridin-5-carbonsäure-pentylester

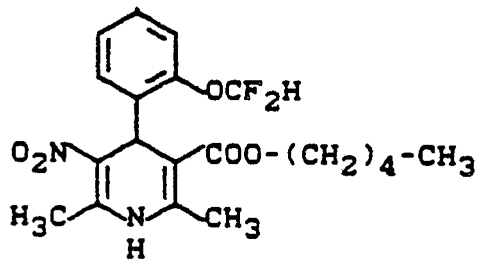

Schmp.: 98 - 100° C

Beispiel 26

4-(2-Difluormethoxyphenyl)-1,4-dihydro-2,6-dimethyl-3-nitro-pyridin-5-carbonsäure-(2-heptyloxy-ethyl)-ester

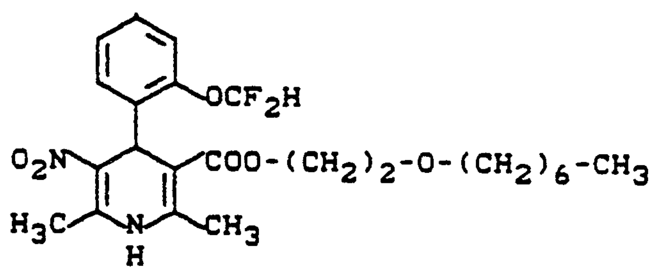

Schmp.: 70 - 73° C

Beispiel 27

4-(2-Difluormethoxyphenyl)-1,4-dihydro-2,6-dimethyl-3-nitro-pyridin-5-carbonsäure-(2-hexyloxy-ethyl)-ester

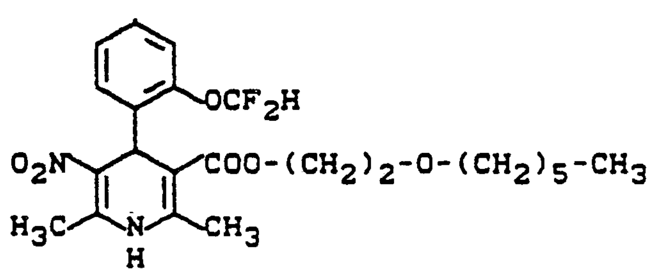

Schmp.: 85° C

Beispiel 28

4-(2-Difluormethoxyphenyl)-1,4-dihydro-2,6-dimethyl-3-nitro-pyridin-5-carbonsäure-(2-pentyloxy-ethyl)-ester

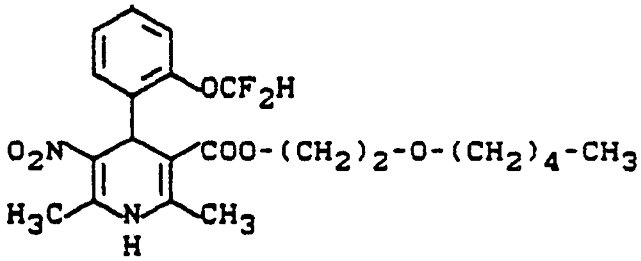

Schmp.: 99 - 101° C

Beispiel 29

4-(2-Difluormethoxyphenyl)-1,4-dihydro-2,6-dimethyl-3-nitro-pyridin-5-carbonsäure-(2-butyloxy-ethyl)-ester

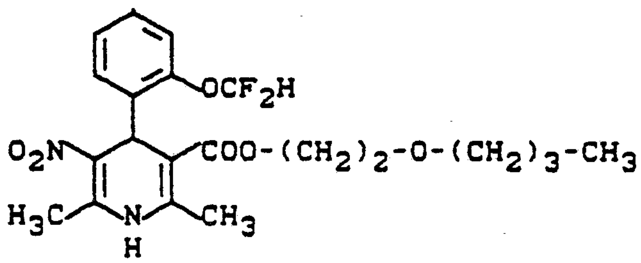

Schmp.: 92 - 93° C

Beispiel 30

4-(2-Difluormethoxyphenyl)-1,4-dihydro-2,6-dimethyl-3-nitro-pyridin-5-carbonsäure-(2-ethylthio-ethyl)-ester

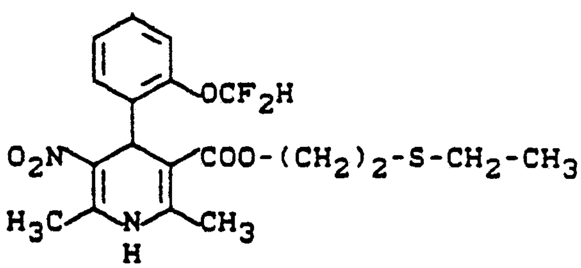

Schmp.: 84 - 85° C

Beispiel 31

4-(2-Difluormethoxyphenyl)-1,4-dihydro-2,6-dimethyl-3-nitro-pyridin-5-carbonsäure-(6-hydroxyhexyl)-ester

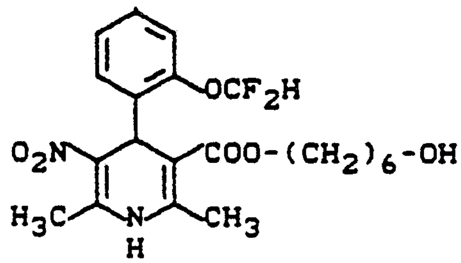

Schmp.: 127° C

Beispiel 32

4-(2-Difluormethoxyphenyl)-1,4-dihydro-2,6-dimethyl-3-nitro-pyridin-5-carbonsäure-(3-methoxycarbonyl-propyl)-ester

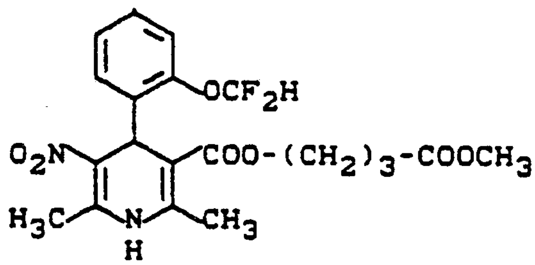

Schmp.: 139 - 140° C

Beispiel 33

4-(2-Difluormethoxyphenyl)-1,4-dihydro-2,6-dimethyl-3-nitro-pyridin-5-carbonsäure-(1-ethoxycarbonyl-ethylester (Diastereomer A)

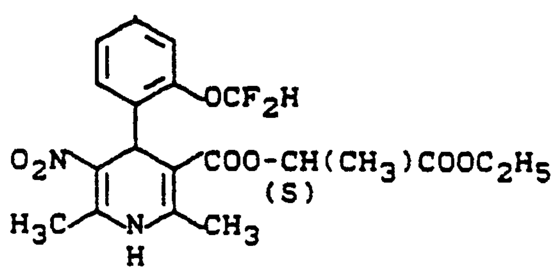

Schmp.: 140 - 141° C

Beispiel 34

4-(2-Difluormethoxyphenyl)-1,4-dihydro-2,6-dimethyl-3-nitro-pyridin-5-carbonsäure-(1-ethoxycarbonyl-ethyl)-ester (Diastereomer B)

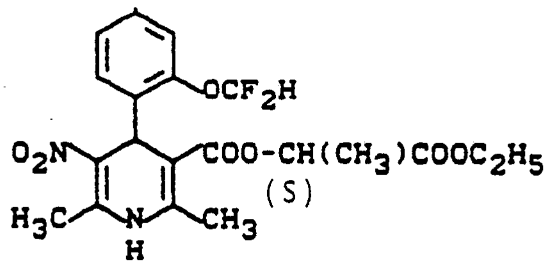

Schmp.: 125 - 126° C

Beispiel 35

4-(2-Difluormethoxyphenyl)-1,4-dihydro-2,6-dimethyl-3-nitro-pyridin-5-carbonsäure-(2-phenoxyethyl)-ester

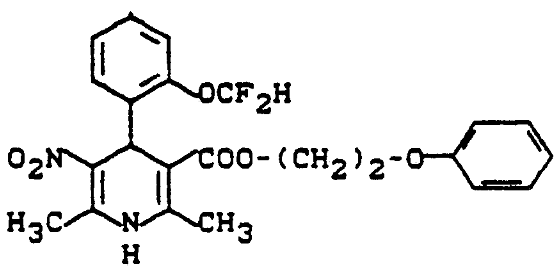

Schmp.: 160 - 162° C

Beispiel 36

4-(2-Difluormethoxyphenyl)-1,4-dihydro-2,6-dimethyl-3-nitro-pyridin-5-carbonsäure-(2-(N-tert -butoxycarbonylamino-2-phenyl)-ethyl)-ester

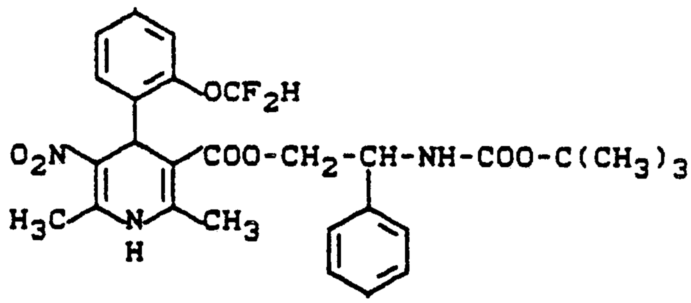

Schmp.: 115 - 119° C

Beispiel 37

4-(2-Difluormethoxyphenyl)-1,4-dihydro-2,6-dimethyl-3-nitro-pyridin-5-carbonsäure-((1,1-dimethyl-2-phenyl)-ethyl)-ester

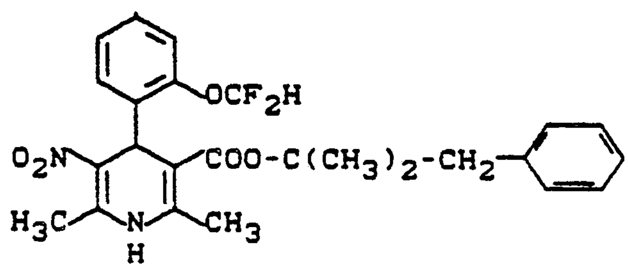

Schmp.: 140 - 141° C

Beispiel 38

4-(2-Difluormethoxyphenyl)-1,4-dihydro-2,6-dimethyl-3-nitro-pyridin-5-carbonsäure-(2-tert.butoxycarbonylamino-4-methyl-pentyl)-ester

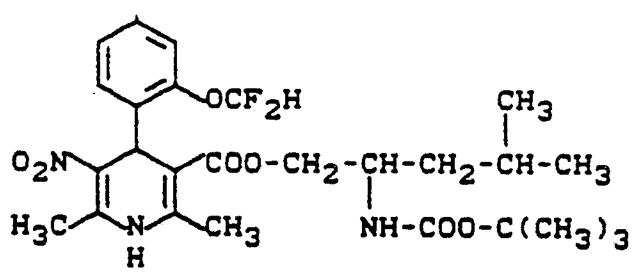

Schmp.: 160 - 162° C

Beispiel 39

4-(2-Difluormethoxyphenyl)-1,4-dihydro-2,6-dimethyl-3-nitro-pyridin-5-carbonsäure-benzylester

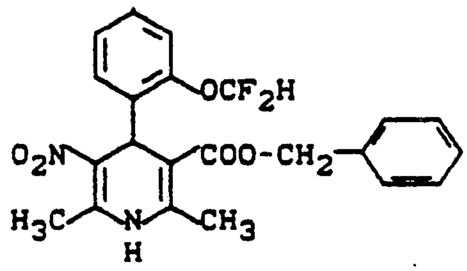

Schmp.: 120 - 122° C

Beispiel 40

4-(2-Difluormethoxyphenyl)-1,4-dihydro-2,6-dimethyl-3-nitro-pyridin-5-carbonsäure-(1-phenylethyl)-ester

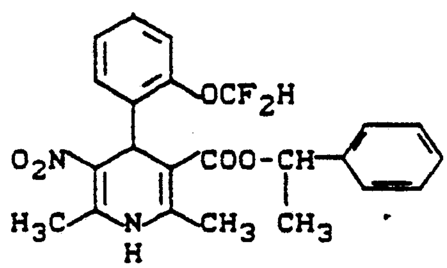

Schmp.: 165 - 169° C

Beispiel 41

4-(2-Difluormethoxyphenyl)-1,4-dihydro-2,6-dimethyl-3-nitro-pyridin-5-carbonsäure-(2-picolyl)-ester

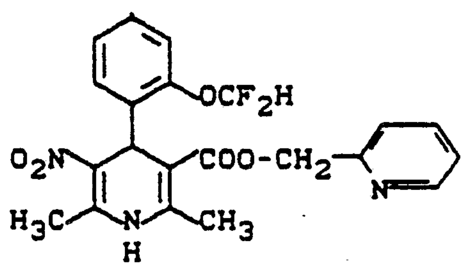

Schmp.: 178 - 180° C

Beispiel 42

4-(2-Difluormethoxyphenyl)-1,4-dihydro-2,6-dimethyl-3-nitro-pyridin-5-carbonsäure-cyclopropylmethylester

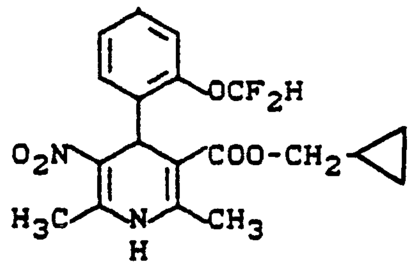

Schmp.: 134 - 136° C

Beispiel 43

4-(2-Difluormethoxyphenyl)-1,4-dihydro-2,6-dimethyl-3-nitro-pyridin-5-carbonsäure-(2-tert.butoxycarbonylaminopropyl)-ester

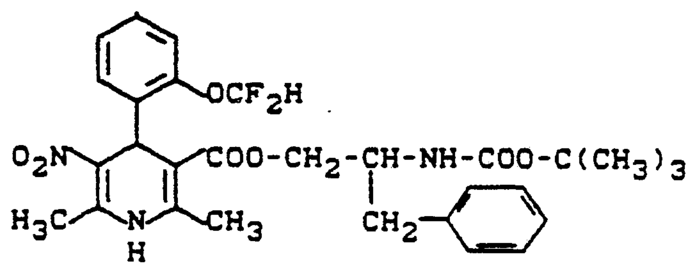

Schmp.: 193° C

Beispiel 44

4-(2-Difluormethoxyphenyl)-1,4-dihydro-2,6-dimethyl-3-nitro-pyridin-5-carbonsäure-1(S)-(1-isopropoxycarbonylethyl)-ester (Diastereomer B)

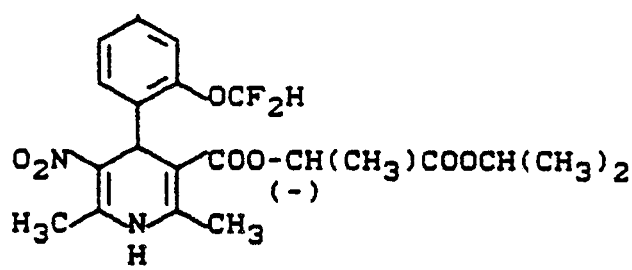

Schmp.: 96 - 98° C

Beispiel 45

4-(2-Difluormethoxyphenyl)-1,4-dihydro-2,6-dimethyl-3-nitro-pyridin-5-carbonsäure-(α-methoxycarbonyl-1-phenyl)-methylester (Diastereomer A)

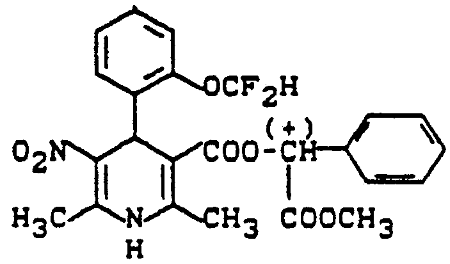

$R_f$ = 0,63 (Methylenchlorid:Essigester 10:1)

Beispiel 46

4-(2-Difluormethoxyphenyl)-1,4-dihydro-2,6-dimethyl-3-nitro-pyridin-5-carbonsäure-(α-methoxycarbonyl-1-phenyl)-methylester (Diastereomer B)

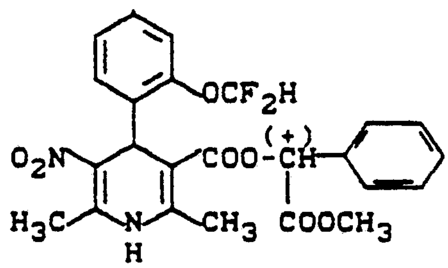

$R_f$ = 0,55 (Methylenchlorid:Essigester 10:1)

Beispiel 47

4-(2-Difluormethoxyphenyl)-1,4-dihydro-2,6-dimethyl-3-nitro-pyridin-5-carbonsäure-(α-methoxycarbonyl-1-phenyl)-methylester (Diastereomer A)

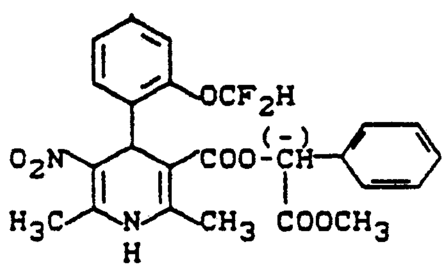

$R_f$ = 0,63 (Methylenchlorid:Essigester 10.1)

Beispiel 48

4-(2-Difluormethoxyphenyl)-1,4-dihydro-2,6-dimethyl-3-nitro-pyridin-5-carbonsäure-(α-methoxycarbonyl-1-phenyl)-methylester (Diastereomer B)

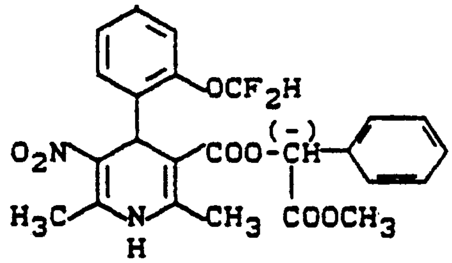

$R_f$ = 0,55 (Methylenchlorid:Essigester 10:1)

Beispiel 49

4-(2-Difluormethoxyphenyl)-1,4-dihydro-2,6-dimethyl-3-nitro-pyridin-5-carbonsäure-allylester

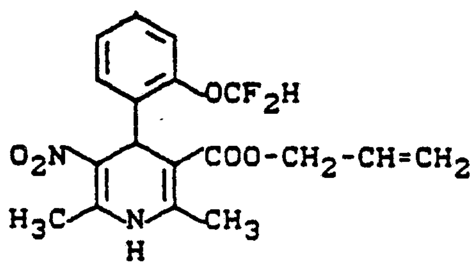

Schmp.: 135 - 137° C

Beispiel 50

4-(2-Difluormethoxyphenyl)-1,4-dihydro-2,6-dimethyl-3-nitro-pyridin-5-carbonsäure-cyclopentylester

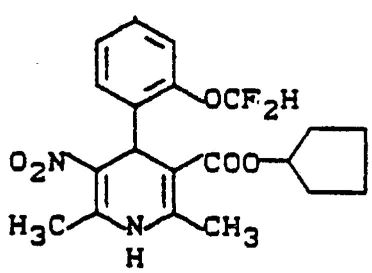

Schmp.: 134 - 136° C

Beispiel 51

4-(2-Difluormethoxyphenyl)-1,4-dihydro-2,6-dimethyl-3-nitro-pyridin-5-carbonsäure-cyclohexylmethylester

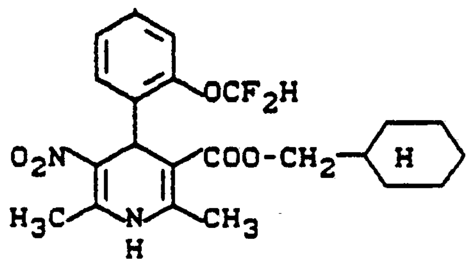

$R_f$ = 0,68 (Toluol:Essigester 1:1)

Beispiel 52

4-(2-Difluormethoxyphenyl)-1,4-dihydro-2,6-dimethyl-3-nitro-pyridin-5-carbonsäure-cyclohexylester

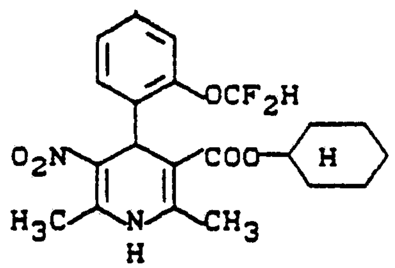

Schmp.: 171 - 173° C

Beispiel 53

4-(2-Difluormethoxyphenyl)-1,4-dihydro-2,6-dimethyl-3-nitro-pyridin-5-carbonsäure-methallylester

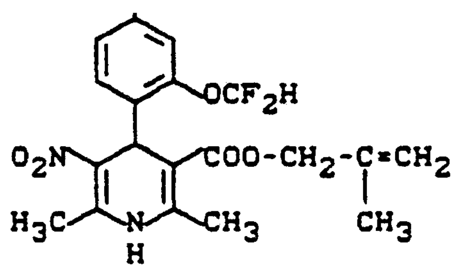

Schmp.: 98 - 100° C

Beispiel 54

4-(2-Difluormethoxyphenyl)-1,4-dihydro-2,6-dimethyl-3-nitro-pyridin-5-carbonsäure-((2-methoxy-2-phenyl)-ethyl)-ester (Diastereomerengemisch)

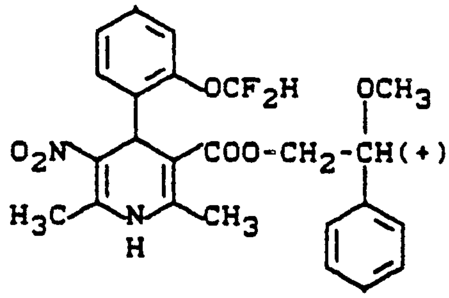

Schmp.:

Beispiel 55

4-(2-Difluormethoxyphenyl)-1,4-dihydro-2,6-dimethyl-3-nitro-pyridin-5-carbonsäure-octylester ((-)-Enantiomer)

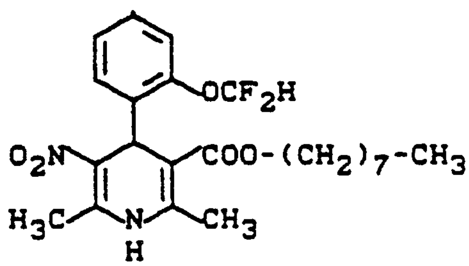

Schmp.: $[\alpha]_{589}^{20}$ = -26.65 (c = 1,076, Chloroform)

Beispiel 56

4-(2-Difluormethoxyphenyl)-1,4-dihydro-2,6-dimethyl-3-nitro-pyridin-5-carbonsäure-(2-phenylethyl)-ester ((-)-Enantiomer)

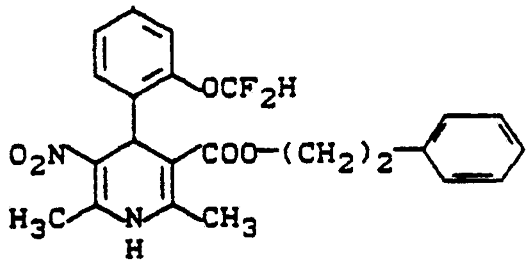

Schmp.: 147 - 149° C

Beispiel 57

4-(2-Difluormethoxyphenyl)-1,4-dihydro-2,6-dimethyl-3-nitro-pyridin-5-carbonsäure-benzylester ((-)-Enantiomer)

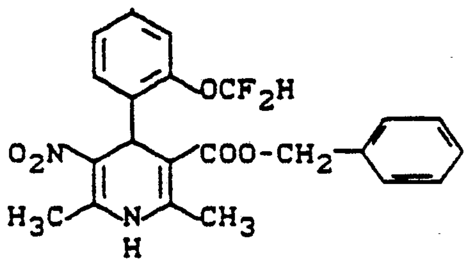

$R_f$ = 0,51 (Toluol/Essigester 2:1)
Schmp.: $[\alpha]_{589}^{20}$ = -49,9 (Chloroform)

Beispiel 58 (analog Beispiele 1-3)

4-(2-Difluormethoxyphenyl)-1,4-dihydro-2,6-dimethyl-3-nitro-pyridin-5-carbonsäure-(10-hydroxy-decylester)

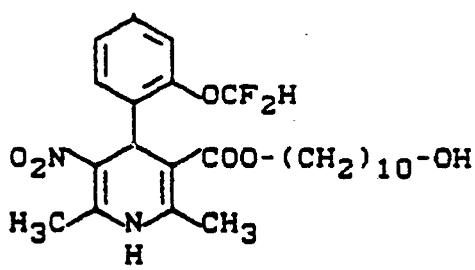

Schmp.: 98° C

Beispiel 59

4-(2-Difluormethoxyphenyl-1,4-dihydro-2,6-dimethyl-3-nitro-pyridin-5-carbonsäure-(10-hydroxy-decylester)

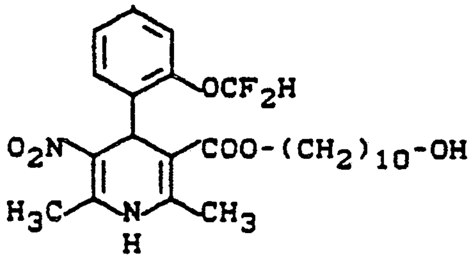

(-)-Enantiomer

$[\alpha]^{20}_{589}$ = 27,92 (Chloroform)
Schmp.: 123° C

Beispiel 60

4-(2-Trifluormethoxyphenyl)-1,4-dihydro-2,6-dimethyl-3-nitro-pyridin-5-carbonsäure-heptylester

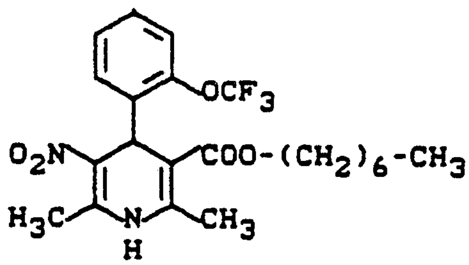

$R_f$ =

Beispiel 61

4-(2-Trifluormethoxyphenyl)-1,4-dihydro-2,6-dimethyl-3-nitro-pyridin-5-carbonsäureoctylester

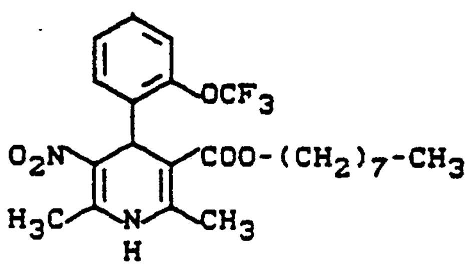

9,5 g (50 mmol) 2-Trifluormethoxybenzaldehyd werden in 75 ml Ethanol mit 10,65 g (50 mmol) β-Aminocrotonsäureoctylester, 9 g (87,5 mmol) Nitroaceton und 3 ml (50 mmol) Essigsäure versetzt und 4 Stunden gekocht. Es wird abgekühlt und eingeengt. Der ölige Eindampfrückstand wird in Essigester aufgenommen, mit Wasser, Natriumhydrogencarbonatlösung und wieder mit Wasser gewaschen, getrocknet und eingeengt. Das erhaltene Öl wird über eine 600 ml Kieselgel-Säule mit Toluol/Essigester gereinigt. Die sauberen Fraktionen werden vereinigt, eingeengt und mit Ether kristallisiert. Es wird abgesaugt und mit Ether gewaschen. Man erhält 6,7 g (28,5 % der Theorie) gelbe Kristalle vom Schmelzpunkt 120-122° C.

Beispiel 62

1,4-Dihydro-2,6-dimethyl-3-nitro-4-(2-trifluormethoxy-phenyl)-pyridin-5-carbonsäure-n-butylester

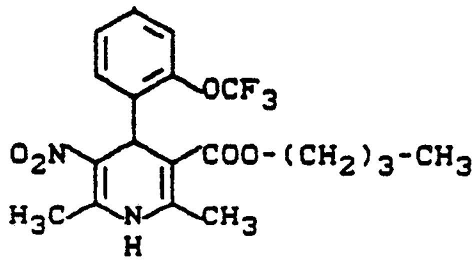

20 mmol = 2,75 g 2-Trifluormethoxybenzyliden-nitroaceton werden in 15 ml Ethanol mit 10 mmol = 1,53 g β-Aminocrotonsäure-n-butylester und 0,6 mmol Eisessig 4 Stunden gekocht. Es wird abgekühlt und eingeengt. Der Eindampfrückstand wird in Essigester aufgenommen, mit Wasser, Natriumhydrogencarbonatlösung und wieder Wasser gewaschen, getrocknet und eingeengt. Das erhaltene Öl wird über eine Kieselgelsäure mit Toluol/Essigester-Gemischen gereinigt. Die sauberen Fraktionen werden eingeengt, der Eindampfrückstand mit Hexan/Ether kristallisiert, abgesaugt und mit Hexan gewaschen. Man erhält 3,2 g (77,3 % der Theorie) gelbe Kristalle vom Schmelzpunkt 122° C.

Analog den Beispielen 64 und 65 wurden hergestellt:

Beispiel 63

4-(2-Trifluormethoxyphenyl)-1,4-dihydro-2,6-dimethyl-3-nitro-pyridin-5-carbonsäure-n-dodecylester

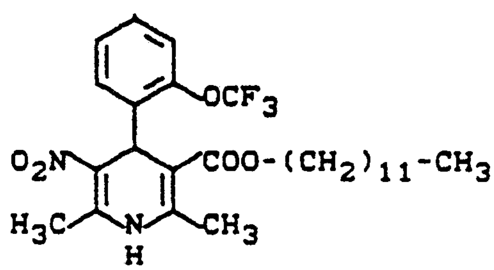

Schmp.: 109° C

Beispiel 64

4-(2-Trifluormethoxyphenyl)-1,4-dihydro-2,6-dimethyl-3-nitro-pyridin-5-carbonsäure-(2-(2-pyridyl)-ethyl)ester

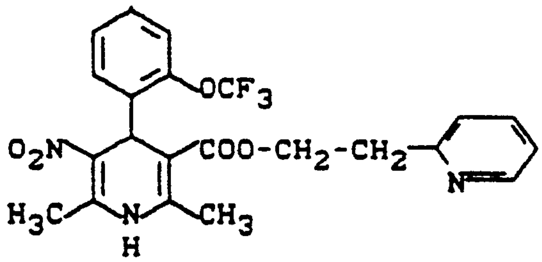

Schmp.: 170° C

Beispiel 65

4-(2-Trifluormethoxyphenyl)-1,4-dihydro-2,6-dimethyl-3-nitro-pyridin-5-carbonsäure-(3,3-dimethylbutyl)-ester

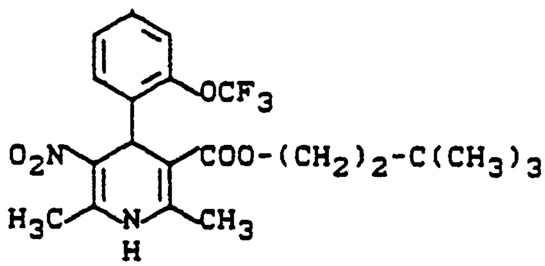

Schmp.: 112° C

Beispiel 66

4-(2-Trifluormethoxyphenyl)-1,4-dihydro-2,6-dimethyl-3-nitro-pyridin-5-carbonsäure-(2-acetoxyethyl)-ester

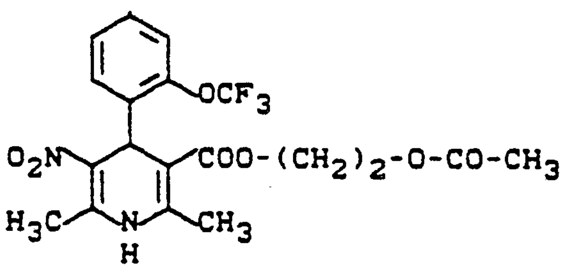

Schmp.: 113° C

Beispiel 67

4-(2-Trifluormethoxyphenyl)-1,4-dihydro-2,6-dimethyl-3-nitro-pyridin-5-carbonsäure-isopropylester

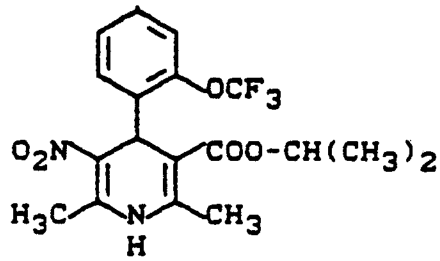

Schmp.: Harz

Beispiel 68

4-(2-Trifluormethoxyphenyl)-1,4-dihydro-2,6-dimethyl-3-nitro-pyridin-5-carbonsäure-(2-cyanoethyl)-ester

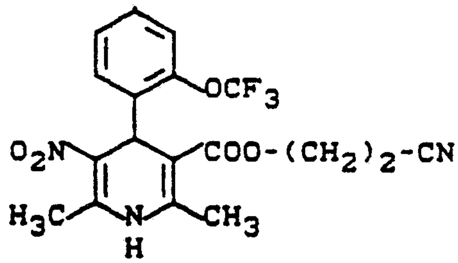

Schmp.: Harz

Beispiel 69

4-(2-Trifluormethoxyphenyl)-1,4-dihydro-2,6-dimethyl-3-nitro-pyridin-5-carbonsäure-(2-(N-benzyl-N-methylamino)-ethyl)-ester

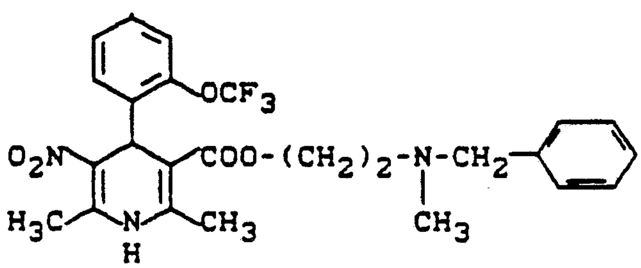

Schmp.: Harz

Beispiel 70

4-(2-Trifluormethoxyphenyl)-1,4-dihydro-2,6-dimethyl-3-nitro-pyridin-5-carbonsäure-(2-(N-piperidinyl)-ethyl)-ester

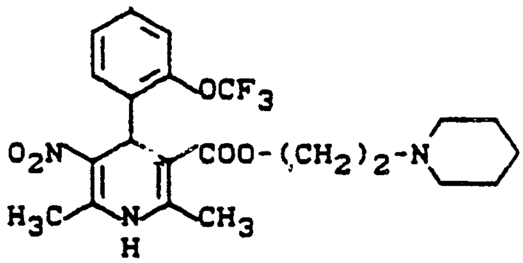

Schmp.: 150° C

Beispiel 71

4-(2-Trifluormethoxyphenyl)-1,4-dihydro-2,6-dimethyl-3-nitro-pyridin-5-carbonsäure-nonylester

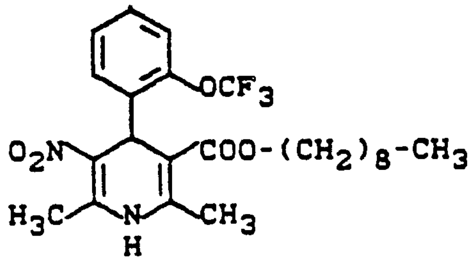

$R_f$ =

Beispiel 72

4-(2-Trifluormethoxyphenyl)-1,4-dihydro-2,6-dimethyl-3-nitro-pyridin-5-carbonsäure-hexadien-2,4-ylester ((-)Enantiomer)

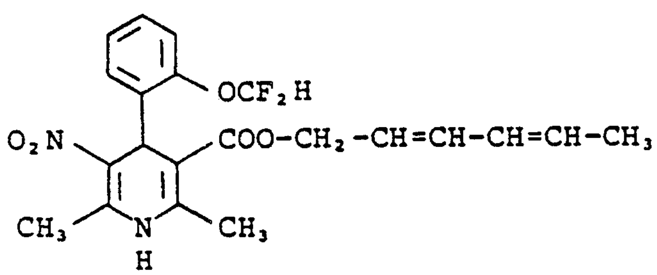

Schmp.: 162° C

Beispiel 73

4-(2-Difluormethoxyphenyl)-2,4-dihydro-2,6-dimethyl-3-nitropyridin-5-carbonsäure-cinnamylester ((-)-Enantiomer)

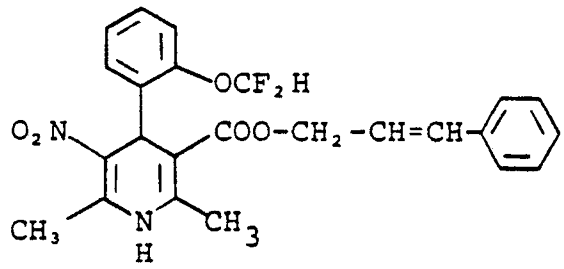

Schmp.: 218 °C

Beispiel 74

4-(2-Difluormethoxyphenyl)-1,4-dihydro-2,6-dimethyl-3-nitropyridin-5-carbonsäure-(2-(N-benzyl-N-methyl)-aminoethyl)-ester ((-)-Enantiomer)

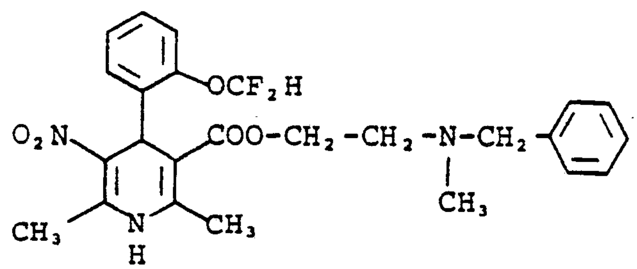

Schmp.: 109 °C

Beispiel 75

4-(2-Difluormethoxyphenyl)-1,4-dihydro-2,6-dimethyl-3-nitropyridin-5-carbonsäure-(2-(N-benzyl)-aminoethyl)-ester ((-)-Enantiomer)

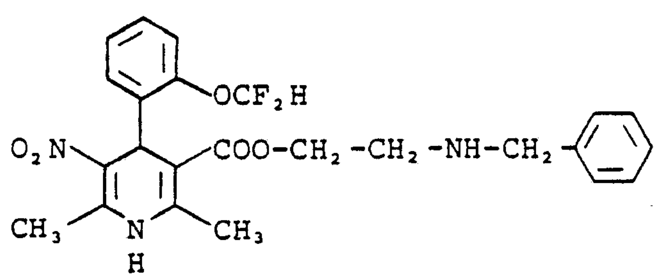

Schmp.: 141 °C

Beispiel 76

4-(2-Difluormethoxyphenyl)-1,4-dihydro-2,6-dimethyl-3-nitro-pyridin-5-carbonsäure-(1,1-diethylmethyl)ester

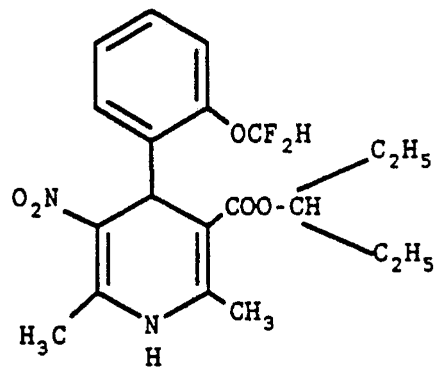

Schmelzpunkt: Öl

**Ansprüche**

1. Fluormethoxyphenyl-dihydropyridine der allgemeinen Formel (I)

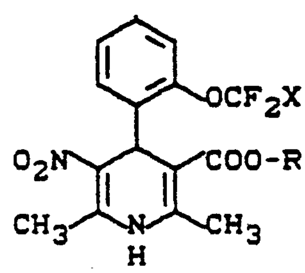

(I)

in welcher
X für Wasserstoff oder Fluor steht und
R - für geradkettiges, verzweigtes oder cyclisches Alkyl oder Alkenyl mit 2 bis 12 Kohlenstoffatomen steht, das in der Kette durch 1 bis 2 Sauerstoff- und/oder Schwefelatome unterbrochen sein kann, und das ein- oder mehrfach gleich oder verschieden substituiert sein kann
a) durch Hydroxy, Aryl mit 6 bis 12 Kohlenstoffatomen, Aryloxy mit 6 bis 12 Kohlenstoffatomen, wobei der Arylrest wiederum durch Alkyl mit bis zu 4 Kohlenstoffatomen, Alkoxy mit bis zu 4 Kohlenstoffatomen oder Halogen substituiert sein kann, oder
b) durch Carboxy, Alkoxycarbonyl mit bis zu 6 Kohlenstoffatomen, Pyridyl, Piperidyl, Pyrimidyl, Acyloxy mit bis zu 7 Kohlenstoffatomen, Sulfamoyl, Carbamoyl, Halogen, Cyano oder
c) durch eine Aminogruppe, wobei die Aminogruppe ein oder zwei gleiche oder verschiedene Substituenten der Reihe Alkyl mit bis zu 6 Kohlenstoffatomen, Benzyl, Phenyl oder Acyl mit biz zu 7 Kohlenstoffatomen tragen kann
in Form von Racematen und von optisch reinen Formen und deren Salze.

2. Verbindungen der allgemeinen Formel (I) gemäß Anspruch 1, in welcher
X - für Wasserstoff oder Fluor steht, und
R - für geradkettiges, verzweigtes oder cyclisches Alkyl mit 2 bis 12 Kohlenstoffatomen steht, das durch Sauerstoff und/oder Schwefel in der Kette unterbrochen sein kann, und das ein- bis zweimal gleich oder verschieden substituiert sein kann durch Hydroxy, Cyano, Phenyl, durch gegebenenfalls durch Methoxy, Methyl, Fluor oder Chlor substituiertes Phenoxy, durch Alkoxycarbonyl mit bis zu 6 Kohlenstoffatomen, durch Pyridyl, Piperidyl, Acetyloxy, Benzoyloxy oder durch eine Aminogruppe, wobei die Aminogruppe ein bis zwei gleiche oder verschiedene Substituenten der Reihe Alkyl mit bis zu 4 Kohlenstoffatomen, Benzyl oder Acetyl tragen kann, oder
- fur geradkettiges oder verzweigtes Alkenyl mit bis zu 10 Kohlenstoffatomen steht
und deren Salze.

3. Verbindungen der allgemeinen Formel (I) gemäß Anspruch 1, in welcher
X - für Wasserstoff oder Fluor steht, und
R - für geradkettiges oder verzweigtes Alkyl mit 6 bis 12 Kohlenstoffatomen steht, das in der Kette durch ein Sauerstoffatom oder ein Schwefelatom unterbrochen sein kann und das ein- oder zweifach gleich oder verschieden substituiert sein kann durch Hydroxy, Cyano, Phenyl, Phenoxy, 4-Methoxyphenoxy, Pyridyl, Alkoxycarbonyl mit bis zu 4 Kohlenstoffatomen, Amino oder Benzylmethylamino oder
- für geradkettiges oder verzweigtes Alkenyl mit bis zu 10 Kohlenstoffatomen steht, oder
- für Cyclopropyl, Cyclopentyl, Cyclohexyl, Cyclopropylmethyl, Cyclopentylmethyl oder Cyclohexylmethyl steht
sowie deren Salze.

4. Verbindungen der allgemeinen Formel (I) gemäß Anspruch 1 in Form von Diastereomerengemischen und in ihren stereoisomer einheitlichen Formen und Enantiomeren.

5. Verfahren zur Herstellung von Verbindungen der allgemeinen Formel (I)

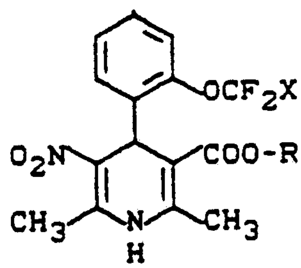

(I)

in welcher
X - für Wasserstoff oder Fluor steht, und
R - für geradkettiges, verzweígtes oder cylcisches Alkyl oder Alkenyl 2 bis 15 Kohlenstoffatomen steht, das in der Kette durch 1 bis 2 Sauerstoff- und/oder Schwefelatome unterbrochen sein kann, und das ein- oder mehrfach gleich oder verschieden substituiert sein kann
a) durch Hydroxy, Aryl mit 6 bis 12 Kohlenstoffatomen, Aryloxy mit 6 bis 12 Kohlenstoffatomen, wobei der Arylrest wiederum durch Alkyl mit bis zu 4 Kohlenstoffatomen, Alkoxy mit bis zu 4 Kohlenstoffatomen oder Halogen substituiert sein kann, oder
b) durch Carboxy, Alkoxycarbonyl mit bis zu 6 Kohlenstoffatomen, Pyridyl, Piperidyl, Pyrimidyl, Acyloxy mit bis zu 7 Kohlenstoffatomen, Sulfamoyl, Carbamoyl, Halogen, Cyano oder
c) durch eine Aminogruppe, wobei die Aminogruppe ein oder zwei gleiche oder verschiedene Substituenten der Reihe Alkyl mit bis zu 6 Kohlenstoffatomen, Benzyl, Phenyl oder Acyl mit bis zu Kohlenstoffatomen tragen kann
in Form von Racematen und von optisch reinen Formen und deren Salze,
dadurch gekennzeichnet, daß man
[A] Aldehyde der Formel (II)

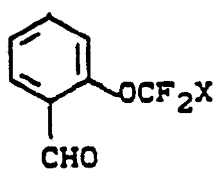

(II)

in dem X die oben angegebene Bedeutung hat,
und Nitroaceton der Formel (III)

$H_3C\text{-}CO\text{-}CH_2\text{-}NO_2$ (III)

oder deren Knoevenagel-Kondensationsprodukt der Formel (IV)

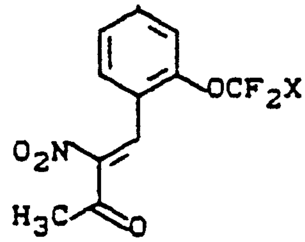

(IV)

in dem X die oben angegebene Bedeutung hat, und Aminocrotonsäureester der allgemeinen Formel (V)

$H_3C-\underset{NH_2}{\underset{|}{C}}=CH-COOR$ (V)

in welcher R die oben angegebene Bedeutung hat, gegebenenfalls in Anwesenheit inerter Lösemittel umsetzt, oder indem man

[B] Dihydropyridin-Derivate der Formel (VI)

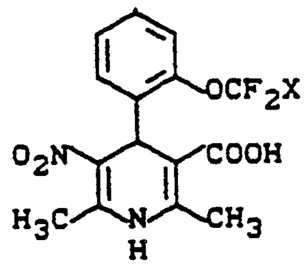

(VI)

in dem X die oben angegebene Bedeutung hat, mit Alkoholen der Formel (VII)

R-OH (VII)

in welcher R die oben angegebene Bedeutung hat, nach bekannten Methoden, gegebenenfalls über ein reaktives Säurederivat, umsetzt, oder indem man

[C] Aldehyde der Formel (II) und Acetessigester der Formel (VIII)

$H_3C-CO-CH_2-COOR$ (VIII)

bzw. deren Knoevenagel-Kondensationsprodukte der Formel (IX)

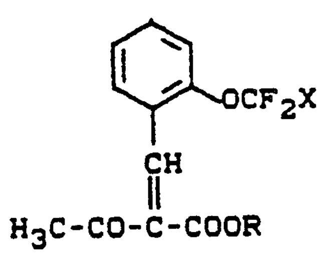

(IX)

in denen X und R die oben angegebenen Bedeutungen haben, mit Nitroaceton und Ammoniumsalzen wie z.B. Ammoniumacetat, gegebenenfalls in Anwesenheit inerter

Lösemittel, umsetzt, und die erhaltenen Dihydropyridine gegebenenfalls nach üblichen Methoden in ihre optisch aktiven Isomere überführt.

6. Verfahren zur Herstellung von Verbindungen der allgemeinen Formel (I) in enantiomerenreiner Form, dadurch gekennzeichnet, daß man Diastereomerengemische von Dihydropyridinen der allgemeinen Formel (X)

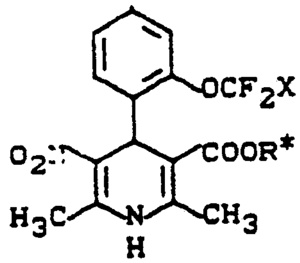

(X)

in welcher
X die oben angegebene Bedeutung hat, und
R* einen optisch aktiven Esterrest darstellt,
durch Kristallisation, Chromatographie oder Craig-Verteilung in die einzelnen Diastereomeren trennt, anschließend die enantiomerenreinen Carbonsäuren der Formel XI

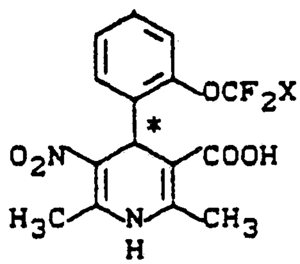

(XI)

herstellt,
und dann diese gegebenenfalls durch Veresterung mit Alkoholen der allgemeinen Formel (VII) in die entsprechenden enantiomerenreinen Dihydropyridine der Formel (I) umsetzt.

7. Verbindungen der allgemeinen Formel (I) gemäß Anspruch 1 zur Verwendung bei der Bekämpfung von Krankheiten.

8. Arzneimittel enthaltend minestens eine Verbindung der allgemeinen Formel (I) gemäß Anspruch 1.

9. Verfahren zur Herstellung von Arzneimitteln, dadurch gekennzeichnet, daß man Verbindungen der allgemeinen Formel (I) gemäß Anspruch 1 gegebenenfalls unter Verwendung von üblichen Hilfs- und Trägerstoffen in eine geeignete Applikationsform überführt.

10. Verwendung von Verbindungen der allgemeinen Formel (I) gemäß Anspruch 1 bei der Herstellung von Arzneimitteln mit positiv inotroper Wirkung.